# EUROPEAN PATENT APPLICATION

(11) **EP 4 112 627 A1**
(43) Date of publication of application: **04.01.2023**
(21) Application number: 21760873.6
(22) Date of filing: 23.02.2021
(51) Int. Cl.: C07F 9/38, A61K 49/06

(54) **MRI CONTRAST AGENT, PREPARATION METHOD THEREFOR AND APPLICATION THEREOF**

(30) Priority: 25.02.2020 CN 202010114947
(71) Applicant: Guangzhou Pinglan Medical Technology Co., Ltd., Guangzhou City, Guangdong 510530 (CN)
(72) Inventor: ZHU, Jiang, Guangzhou City, Guangdong 510530 (CN); ZHONG, Lei, Guangzhou City, Guangdong 510530 (CN); CHEN, Keyu, Guangzhou City, Guangdong 510530 (CN); NIE, Yuting, Guangzhou City, Guangdong 510530 (CN); ZHANG, Junli, Guangzhou City, Guangdong 510530 (CN); XIA, Qian, Guangzhou City, Guangdong 510530 (CN); SHEN, Chengyi, Guangzhou City, Guangdong 510530 (CN); LEI, Jun, Guangzhou City, Guangdong 510530 (CN); ZHANG, Xiaoming, Guangzhou City, Guangdong 510530 (CN); MI, Qingning, Guangzhou City, Guangdong 510530 (CN)
(74) Representative: Henderson, Helen Lee
(86) International application number: PCT/CN2021/077399
(87) International publication number: WO 2021/169934

(57) **Abstract**

The present disclosure relates to a magnetic resonance imaging (MRI) contrast agent and a preparation method and a use thereof, which belong to the technical field of Magnetic Resonance Imaging contrast agent. The MRI contrast agent is prepared by the compound having a structure of formula I; and it also may comprise the compound having a structure of formula II or a pharmaceutically acceptable salt thereof, wherein M₁ is a divalent ion or a trivalent ion of a paramagnetic metal selected from Mn, Fe, Eu, or Dy ; M₂ is selected from Na⁺, K⁺ or meglumine cation; when M₁ is a divalent ion, a is 2; when M₁ is a trivalent ion, a is 3. The MRI contrast agent has good water solubility, high relaxivity, and low toxic and side effect.

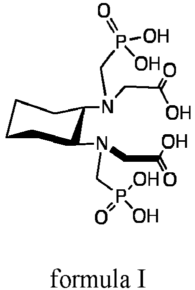

## Description

### FIELD OF TECHNOLOGY

The present disclosure relates to the technical field of Magnetic Resonance Imaging contrast agent, particularly, it relates to a magnetic resonance imaging contrast agent and a preparation method and a use thereof.

### BACKGROUND

Magnetic Resonance Imaging (MRI) is an application of nuclear magnetic resonance technology in the medical field. Compared with other medical imaging technologies, it has the advantages such as high soft-tissue contrast, multi-parameter mapping and multi-sequence imaging, and non-ionizing radiation damage. At present, nearly 50% of enhanced magnetic resonance imaging scans in clinic are performed with magnetic resonance imaging contrast agents based on the rare earth metal, Gadolinium (Gd³⁺). In the human body, Gadolinium is an exogenous metal. Free Gd³⁺ is highly toxic, distributed in bones and liver in body, and can rapidly lead to liver necrosis. Since the Food and Drug Administration (FDA) approved Magnevist^{®} (Bayer) in 1988, gadolinium-based contrast agents (GBCA) have been clinically used for 30 years, and their safety problems, such as Nephrogenic Systemic Fibrosis (NSF) and Gadolinium Retention in the brain, have become increasingly obvious.

Since 2006, the Food and Drug Administration (FDA) of the United states has continuously found that patients suffering from renal insufficiency (glomerular filtration rate < 30 mL/min/ 1.73 m²) may develop NSF symptoms - overall skin sclerosis, because some low-stability gadolinium-based contrast agents cannot be completely and timely cleared from the body after use. Some severe patients may develop damages on organs. Currently, there is no effective treatment method to NSF.

In recent years (since 2014), there is an evidence that GBCA can be accumulated for a long period after the last examination in the brains of some patients who have been examined by contrast-enhanced MRI with GBCA for four or more times. A researcher from the Mayo clinic finds that GBCA Retention can be in the brains of some deceased patients who have been examined by contrast-enhanced MRI with GBCA for several times, and there is dose-effect relationship, without association of renal function, age, and time interval between first exposure and death.

In conclusion, GBCA has potentially huge side effects and safety problems, such as NSF caused by Gd³⁺ based contrast agent and brain retention, which have become an urgent problem to be solved in clinic.

### SUMMARY

Based on this, it is necessary to address the potentially huge side effects caused by the Gd-based magnetic resonance imaging contrast agent and provide a compound having a structure of formula I or a pharmaceutically acceptable salt thereof, wherein the compound having a structure of formula I has a suitable oil-water partition coefficient, and can be used for preparing a magnetic resonance imaging contrast agent, and can provide an important structural basis for further preparation of non-Gd based magnetic resonance imaging contrast agent.

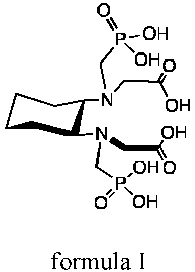

In one example, the pharmaceutically acceptable salt is selected from a compound having a structure of formula III or IV: 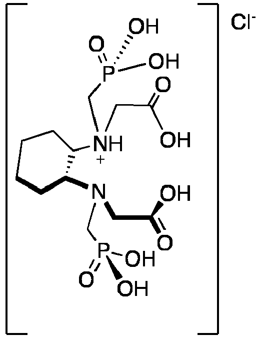 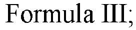 or

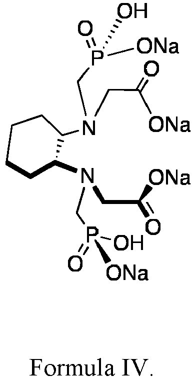

It should be understood that the pharmaceutically acceptable salt can be in other forms, which may be regulated according to the method of hydrolysis during preparation.

It is another aspect of the present disclosure to provide a method of preparing a compound having a structure of formula I, and the preparation method is shown in the following synthetic scheme: 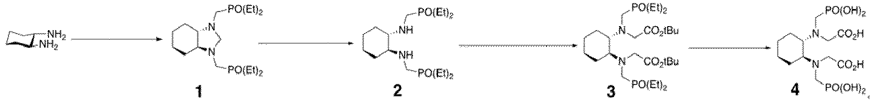
S1: condensing paraformaldehyde and phosphinate through a Kabachnick-Fields reaction to obtain Compound 1 with a raw material (cyclohexanediamine);
S2: hydrolyzing the Compound 1 in an acid to obtain Compound 2;
S3: N-alkylating the Compound 2 to obtain Compound 3;
S4: hydrolyzing the Compound 3 to obtain Compound 4, i.e. the compound having a structure of formula I.

It should be understood that, the configuration of the raw material (cyclohexanediamine) can be selected according to the target compound, preferably the raw material is trans-cyclohexanediamine (trans-CDTA), which has a better stability.

In one example, the preparation method comprises steps of:
S1: adding cyclohexanediamine, diethyl phosphite and paraformaldehyde into a solvent (tetrahydrofuran), mixing them and refluxing to obtain Compound 1;
S2: adding methanol to the compound obtained in the S1 and reacting in a high concentration acid to obtain Compound 2. The above high concentration acid is 2 to 4 M HCl, preferably 3M HCl, or other organic or inorganic acids that can provide the same acidic conditions (H⁺ concentration is 2 to 4M).
S3: dissolving the compound obtained in the S2 into acetonitrile, adding KI, tert-butyl bromoacetate and alkali, and then reacting to obtain Compound 3. Preferably, the alkali is selected from a group consisting of N,N-diisopropylethylamine (DIPEA) or triethylamine (TEA).
S4: dissolving the compound obtained in the S3 into an acid solution, heating with reflux, and removing a protecting group to further obtain Compound 4, i.e. the compound having a structure of formula I. As this step is a hydrolysis reaction, it should be understood that suitable acids and their concentrations for the acid solution can be adjusted according to specific reaction conditions, such as 4 to 8 M HCl, preferably 6M HCl, or other organic or inorganic acids that can provide the same acidic conditions (H⁺ concentration is 4 to 8M).

In one example, the preparation method comprises the steps of:
S1: adding anhydrous tetrahydrofuran, cyclohexanediamine, diethyl phosphite and paraformaldehyde, mixing them and refluxing, then removing tetrahydrofuran, followed by adding dichloromethane and water to perform extraction, remaining organic phase, washing, then drying and filtering to remove dichloromethane, thereby obtaining Compound 1;
S2: adding methanol and concentrated hydrochloric acid to the compound obtained in S1 and reacting, removing methanol after reaction, and then adding water, followed by adding alkali to neutralize acids, filtering and adding dichloromethane to perform extraction, remaining organic phase, washing, then drying and filtering to remove dichloromethane, thereby obtaining Compound 2, wherein a mass fraction of HCl in the concentrated hydrochloric acid is 36%~38%.
S3: dissolving the compound obtained inS2 in anhydrous acetonitrile, then adding KI, N,N-diisopropylethylamine, and tert-butyl bromoacetate and reacting; after reaction, removing acetonitrile, followed by adding ethyl acetate and water to perform extraction, remaining organic phase, washing, then drying and filtering to remove ethyl acetate, thereby obtaining Compound 3.
S4: dissolving the compound obtained in the S3 into hydrochloric acid, heating with reflux, and after reaction removing liquid, then adding water for dissolution, followed by adding acetonitrile, and then precipitating a white solid, filtering to obtain a solid, Compound 4, i.e. the compound having a structure of formula I.

In one example, S1 is performed as follows: mixing anhydrous tetrahydrofuran, cyclohexanediamine, diethyl phosphite and part of paraformaldehyde and heating with reflux at 92 to 98 °C for 3 to 5 hours, and adding rest of paraformaldehyde in 3 to 5 times during reflux until the reaction is completed, performing rotary evaporation under decreased pressure to remove tetrahydrofuran, adding dichloromethane and water to perform extraction, remaining organic phase, washing by water or saturated saline solution, drying the organic phase with anhydrous sodium sulfate, filtering, and performing rotary evaporation under decreased pressure to remove dichloromethane to obtained Compound 1, wherein a molar ratio of cyclohexenediamine, diethyl phosphite and paraformaldehyde is 1 : (1.8 to 2.2) :(2.8 to 3.2).

In one example, S2 is performed as follows: adding methanol and concentrated hydrochloric acid to the compound obtained in the S1 and reacting at 48 to 52°C for 6 to 12 hours, performing rotary evaporation after reaction to remove methanol under decreased pressure, adding water followed by adding alkali to neutralize acids until pH being at 6.8 to 7.2, filtering, and adding dichloromethane to perform extraction, remaining organic phase, washing by saturated saline solution, adding anhydrous sodium sulfate for drying, filtering, and performing rotary evaporation under decreased pressure to remove dichloromethane, to obtain Compound 2, wherein a volume ratio of methanol and concentrated hydrochloric acid is 8: (2 to 4).

In one example, S3 is performed as follows: dissolving the compound obtained in S2 into anhydrous acetonitrile, adding catalysts, i.e. KI, tert-butyl bromoacetate and N,N-diisopropylethylamine or triethylamine, and reacting at 68 to 72 °C for 5 to 7 hours, filtering to remove precipitate after reaction, performing rotary evaporation under decreased pressure to remove acetonitrile, adding ethyl acetate and water to perform extraction, remaining organic phase, adding saturated saline solution for washing, then adding anhydrous sodium sulfate for drying, filtering, performing rotary evaporation under decreased pressure to remove ethyl acetate, purifying by a silica gel column to obtain Compound 3.

In one example, S4 is performed as follows: dissolving the compound obtained in the S3 into 5.5 to 6.5 mol/L hydrochloric acid solution, heating with reflux for 6 to 12 hours, performing rotary evaporation under decreased pressure after reaction to remove liquids, then adding water to dissolve residues and cooling after complete dissolution, adding acetonitrile and stirring well, placing until a white solid is then precipitated, filtering and drying to obtain Compound 4.

It is another aspect of the present disclosure to provide a use of the compound having a structure of formula I or a pharmaceutically acceptable salt thereof in a preparation of a magnetic resonance imaging contrast agent. The contrast agent has a good water solubility, high relaxivity, and low toxic and side effects.

In one example, the compound having a structure of formula I is used as a ligand in a preparation of magnetic resonance imaging contrast agent.

It is another aspect of the present disclosure to provide a compound having a structure of formula II or a pharmaceutically acceptable salt thereof: 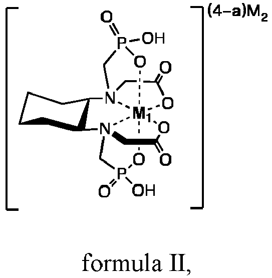 wherein
M₁ is a divalent ion and selected from Mn, Fe, Eu or Dy, or M₁ is a trivalent ion and selected from Mn, Fe, Eu or Dy;
M₂ is selected from Na⁺, K⁺ or meglumine cation;
When M₁ is a divalent ion, a is 2; when M₁ is a trivalent ion, a is 3.

The above compound or pharmaceutically acceptable salt thereof can be used as a non-gadolinium-based paramagnetic metal complex, a magnetic resonance imaging contrast agent, wherein the compound having a structure of formula I is a ligand and Mn, Fe, Eu or Dy are coordinated metal ions. The compound and the salt thereof have the characteristics of good water solubility, high relaxivity and low toxic and side effects, and therefore they can be used as a MRI contrast agent in clinic.

In one example, M₁ is Mn²⁺or Fe ³⁺; M₂ is Na⁺, K⁺ or meglumine cation; a is 2.

Manganese is an essential trace element for human body and is involved in many important biochemical reactions in body. High-spin Mn²⁺ has high paramagnetism and therefore can be used as a paramagnetic metal center for the magnetic resonance imaging contrast agent. According to the characteristics of Mn²⁺ in coordination chemistry, such novel Mn(II) T1 magnetic resonance imaging contrast agent has a similar relaxivity to the commercial gadolinium-based contrast agent, without the risk of Nephrogenic Systemic Fibrosis (NSF), and thus this Mn-based contrast agent can also be used for contrast-enhanced examination in patients suffering from renal insufficiency.

Fe is also an essential trace element for human body, and plays an important role in physiological functions in body, such as hemoglobin transporting oxygen. In general, the human body contains about 4 grams of iron. There is a highly coordinated regulatory system to ensure iron transport and storage in body. The long-term toxicity of Fe³⁺ is much lower than that of Gd³⁺. The high-spin Fe³⁺ complex also has a higher magnetic moment (lower than Mn²⁺ and Gd³⁺), and given the potential safety problem of the gadolinium contrast agent, a corresponding Fe³⁺ complex of the compound having a structure of formula II of the present invention can also be used as Fe-based magnetic resonance imaging contrast agent.

It is another aspect of the present disclosure to provide a method of preparing the compound having a structure of formula II, comprising following steps:
reacting a compound having a structure of formula I or a pharmaceutically acceptable salt thereof with a compound having an M₁ ion, and adding alkali for regulating pH in a range of 6 to 9, thereby obtaining the compound having a structure of formula II.

In one example, the method for preparing the compound having a structure of formula II comprises steps of:
mixing the compound having a structure of formula I with water, adding metallic compounds having Mn, Fe, Eu, or Dy, wherein metal ions of the metallic compounds have a charge of plus 2 or plus 3, regulating pH in a range of 6 to 9, freeze-drying resulting solution to obtain a paramagnetic metal complex.

Preferably, alkali is used for regulating pH in a range of 7 to 8, more preferably 7.3 to 7.4.

In one example, alkali is sodium hydroxide, potassium hydroxide, or meglumine.

It is another aspect of the present disclosure to provide a use of the compound having a structure of formula II in a preparation of a magnetic resonance imaging contrast agent.

It is another aspect of the present disclosure to provide a magnetic resonance imaging contrast agent, comprising the compound having a structure of formula II and a pharmaceutically acceptable excipient.

Compared with the prior art, the present disclosure has the benefits as follows:
The compound having a structure of formula I or pharmaceutically acceptable salt thereof, as a ligand, has a proper oil-water partition coefficient, and can be used for preparing a magnetic resonance imaging contrast agent, and can provide an important structural basis for further preparation of a non-gadolinium based magnetic resonance imaging contrast agent.

The compound having a structure of formula II or pharmaceutically acceptable salt thereof has the characteristics of good water solubility, high relaxivity and low toxic and side effects, and can be used as a MRI contrast agent in clinic, thereby reducing the toxic and side effects of the gadolinium magnetic resonance imaging contrast agent.

### BRIEF DESCRIPTION

Figure 1 depicts a diagram showing crystal structure of Compound 4 in the Example;
Figure 2 depicts a test result of relaxivity of KBR0826 in the Example;
Figure 3 depicts a standard curve of the concentration of Mn ion in the Example;
Figure 4 depicts a pharmacokinetics test result of KBR0826 in the Example;
Figure 5 depicts a test result of excretion kinetics of KBR0826 in the Example;
Figure 6 depicts a hepatotoxicity result of KBR0826 in the Example;
Figure 7 depicts the in vivo image of KBR0826 in rats in the Example; and
Figure 8 depicts the signal intensity of the in vivo image of KBR0826 in rats in the Example.

### DETAILED DESCRIPTION

For better understanding of the present disclosure, the present disclosure will be fully described below with some preferred embodiments. However, the present disclosure can be implemented in many different forms and is not limited to the embodiments described herein. Rather, these embodiments are provided for the purpose of making the disclosed contents of the present disclosure more thorough and complete.

Unless otherwise defined, all technical and scientific terms used herein have the same meaning as those normally understood by one skilled in the art in the technical field that the present disclosure is belonged to. The terms used in the description of the present disclosure herein are only for the purpose of describing specific embodiments, and are not intended to limit the present disclosure.

### Example 1

A ligand, *trans*-cyclohexanediamine-dimethylenephosphono-diacetic acid was prepared, the synthetic scheme was shown as follows:

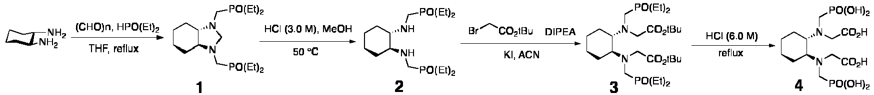

The specific preparation method of the ligand was shown as follows:

### (1) Preparation of Compound 1

cyclohexanediamine (10g, 98%, 85.8 mmol), diethyl phosphite (24.2 g, 98 %, 171.6 mmol) and paraformaldehyde (total amount of 8.31 g, 96%, 266 mmol, about one fifth of paraformaldehyde was added firstly) were added into a 100 mL solvent (anhydrous tetrahydrofuran, THF), heated to 95 °C with reflux, and rest of paraformaldehyde was added in 4 times, refluxed for 4 hours;
After the reaction was completed, the solvent was removed by rotary evaporation under decreased pressure; about 100 mL of dichloromethane and about 100ml of water were respectively added to the residue to perform extraction; then water phase was extracted by dichloromethane for three times (about 20ml per time), organic phase was combined and washed by water and saturated saline solution, respectively, with about 50 mL per time; the organic phase was dried by anhydrous sodium sulfate, filtered, and performed rotary evaporation under decreased pressure to remove dichloromethane, and then a dark purple oily crude product, Compound 1 (27.37 g, yield: 72.8 %), was obtained.

The structural characterization of the Compound 1 is:
¹H -NMR (400 MHz, CDCl₃) δ: 1.20 (d, 4H), 1.35 (t, 12H), 1.72 (s, 2H), 1.93 (d, 2H), 2.16(s, 2H), 2.71 (br, 2H), 3.15 (t, 2H), 3.89 (s, 2H), 4.15(br, 8H);
MS (ESI): [M+H]⁺: calculated value is 427.20, measured value is 427.2.

### (2) Preparation of Compound 2

The Compound 1 (27.1 g, 63.56 mmol) prepared in the step (1) was dissolved into a solution of 120 mL methanol and 45 mL of concentrated hydrochloric acid, reacted at 50 °C overnight; After the reaction was completed, methanol was removed by rotary evaporation, and about 150 mL of water was added to residue followed by neutralizing mixture by NaHCO₃, then redundant NaHCO₃ was filtered and removed; about 100mL dichloromethane was used for performing extraction, the water phase was washed twice again by dichloromethane, the organic phase was combined and washed once by saturated saline solution; anhydrous sodium sulfate was added for drying, and the solution was filtered and dichloromethane was removed by rotary evaporation under decreased pressure, and then a wine red crude product, Compound 2 (11.51 g, yield: 43.7%), is obtained.

The structural characterization of the Compound 2 is:
¹H-NMR (400 MHz, CDCl₃) δ : 0.98 (d, 2H), 1.18 (t, 2H), 1.32 (t, 12H), 1.75 (d, 2H), 2.09 (d, 2H), 2.22 (d, 2H), 2.85 (t, 2H), 3.15 (t, 2H), 4.18 (br, 8H);
MS (ESI): [M+H]⁺: calculated value is 415.20, measured value is 415.2.

### (3) Preparation of Compound 3

the Compound 2 (11.50 g, 27.75 mmol) obtained in step (2) was dissolved into anhydrous acetonitrile (80 mL), and a catalyst, KI (0.46 g, 2.8 mmol), DIPEA (99%, 8.95 g, 69.38mmol), tert-butyl, bromoacetate (98%, 13.53 g, 69.38 mmol) were added successively, and reacted at 70 °C for 6 hours;
after the reaction was completed, precipitate was removed by filtration, rotated to be dried, and then ethyl acetate and water were used for dissolution and extraction; organic phase was washed once by saturated saline solution, then dried by anhydrous sodium sulfate, filtered, rotated under decreased pressure to remove ethyl acetate, and then a crude product of Compound 3 was obtained followed by purifying through a silica gel column to obtain Compound 3 (12.75 g, yield: 71.5%).

The structural characterization of the Compound 3 is:
¹H- NMR (400 MHz, CDCl₃) δ : 1.12 (d, 4H), 1.35 (t, 12H), 1.48 (s, 18H), 1.69 (s, 2H), 2.03 (d, 2H), 2.78 (s, 2H), 3.16(t, 2H), 3.42(br, 2H), 3.52 (d, 2H), 3.65(d, 2H), 4.18 (br, 8H);
MS (ESI): [M+H]⁺: calculated value is 643.34; measured value is 643.4; [M+Na]⁺: calculated value is 665.34; measured value is 665.4

### (4) Preparation of Compound 4

the compound (12.75 g, 19.84 mmol) obtained in the step (3) was dissolved into 6 M hydrochloric acid (100 mL), heated with reflux overnight; after the reaction was completed, the reaction mixture was rotated to be completely dried, followed by adding a little water (about 10 mL) to dissolve residues under heating condition; after complete dissolution, the solution was cooled and 100 mL of acetonitrile was added to the mixture solution which was then stirred and placed until a white solid was precipitated, the solid was then stirred into a powder in the mixed solution, filtered, and washed with a little acetonitrile, then a white powder solid was obtained. After drying a white powder Compound 4 (7.34 g, yield: 88.4 %) was obtained.

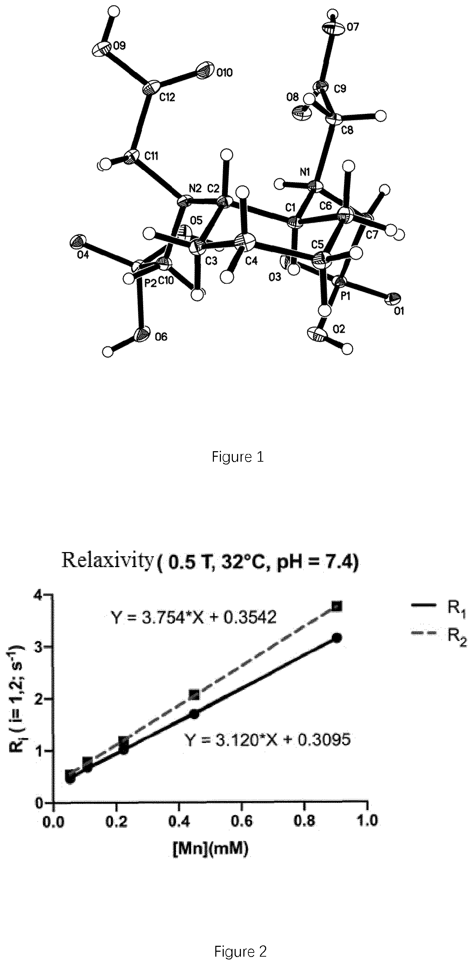

The structural characterization of the Compound 4 is:
¹H-NMR (400 MHz, D₂O)
MS (ESI): [M+Na]⁺: calculated value is 441.09; measured value is 441.0; [M-H]⁺: calculated value is 417.09; measured value is 417.0. The crystal structure of the Compound 4 is shown in Figure 1.

### Example 2

Paramagnetic Mn complex magnetic resonance imaging contrast agent was prepared, comprising the following steps:
the Compound 4 (7.19 g, 17.2 mmol) obtained in the Step (4) of Example 1 was added to 60 mL of pure water, and manganese chloride tetrahydrate (3.33 g, 16.8 mmol) was added under stirring. Solid sodium hydroxide was used for regulating pH in a range of 7.3 to 7.4 to obtain a solution, which was freeze-dried to obtain a solid product of 11.68 g.

MS (ESI): [M+H]⁺: calculated value is 470.0; measured value is 469.9; [M+3H]⁺: calculated value is 472.0; measured value is 472.0.

### Example 3

The following performance tests were done on the paramagnetic Mn - complex magnetic resonance imaging contrast agent (named as KBR0826) obtained in the Example 2:

### I. Relaxivity measurement

The method for measuring the relaxivity comprises the following steps: preparing contrast agent solutions in a gradient concentration (0.054, 0.109, 0.224, 0.451, 0.906 mM, in Hepes buffer, pH = 7.4), and measuring the contrast agent samples at different concentrations by Niumag PQ001 MRI Contrast Agent Analyzer (0.5±0.08T, main frequency of instrument: 21.3MHz; at 32°C) to respectively obtain their *T1* and *T2* relaxation time, and then performing a linear fitting between the relaxation rate, *Rᵢ* (*Rᵢ* = *1*/*Tᵢ, i* = 1, 2) and the contrast agent concentrations to obtain a slope, i.e., relaxivity (*r*ᵢ, *i* = 1, 2). Relaxivity reflects the ability of magnetic resonance imaging contrast agent to alter the relaxation time of water proton and is an important parameter to evaluate the quality of contrast agent. Relaxivity (*rᵢ, i =* 1, 2) is defined as the alteration in the relaxation rate of water proton (1/*Tᵢ*, *i* = 1, 2) by unit concentration of MRI Contrast agent (mmol/L).

The test results are shown in Figure 2, where the abscissa is the molar concentration of Mn²⁺ and the ordinate is 1/T (s⁻¹). At 0.5 T (32°C, pH = 7.4), the relaxivities of KBR0826 are as follows: *r₁* = 3.12 mmol⁻¹s⁻¹, *r₂* = 3.75 mmol⁻¹s⁻¹, *r₂* / *r₁* = 1.2, which correspond to those of Magnvist^{®} (*r₁* = 3.4mmol⁻¹s⁻¹, *r₂* = 4.0 mmol⁻¹s⁻¹).

### II. Pharmacokinetics Study

### Method of Pharmacokinetics Study

### 1. Experimental preparation

Totally 8 SD rats, 3 female rats and 5 male rats, weighed 220 to 240 g, were anesthetized by intraperitoneal injection of 3% pentobarbital sodium (50 mg/kg) and fixed in supine position, followed by removing neck hair and disinfecting routinely for later use.

Catheterization of left carotid artery comprises steps as follows: making a 20 mm longitudinal incision from the 3 mm left side of the median cervical line (the line from the sternum to the mandible) and 7 mm above the sternum; dissecting the skin to expose the subcutaneous tissue, and performing a blunt layer-by-layer dissection until the carotid artery was visualized; separating nerve from its accompanied veins by blunt dissection; ligating distal portion and proximal portion of the heart respectively, wherein the distance between the nodes was 2.5-3.5cm; inserting an indwelling needle into the artery near the node at the distal portion of the heart and fixing with a hemostatic clip, taking the needle out and releasing the node at the proximal portion of the heart; and injecting heparin lithium (150 µg/kg) to perform systemic heparinization in body.

### 2. Collection and treatment of blood samples

Caudal vein was rapidly injected (< 5s) with KBR0826 solution (0.05mmol/mL) at a dose of 0.1mmol/kg. Before injection and within 1 min, 2 min, 3 min, 5 min, 8 min, 13 min, 20min, 30min, and 50 min after the injection, 0.1mL of carotid blood was respectively collected and placed in a 0.5mL EP tube and stored at - 20 °C.

### 3. Concentration measurement of Mn²⁺ in samples by Inductively coupled plasma mass spectrometry (ICP-MS)

### 3.1 Working conditions

ICP-MS was configured with a power of 1150W, a flow rate of argon for atomization gas of 0.95L· min⁻¹, a flow rate of argon for auxiliary gas of 1.2 L· min⁻¹, a flow rate of argon for plasma gas of 18L· min⁻¹, pump speed of 20r· min⁻¹, an analytic mode being standard mode, repetition sampling in triple.

### 3.2 Establishment of standard (calibration) curve

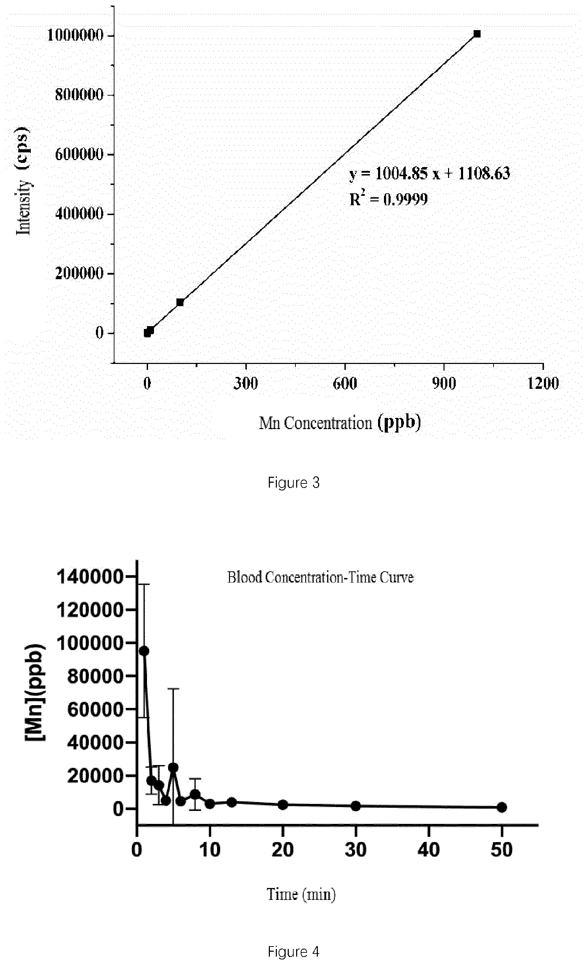

A set of standard solution was prepared with standard reagents (PerkinElmer, 10µg/mL (1000 ppb): Ag, Al, As, Cs, Cu, Sr, Se, Mn, etc.) at different concentrations, i.e. at 1.0 ppb, 10 ppb, 50 ppb, 100 ppb, 200 ppb and 1000 ppb, to establish a standard curve (as shown in Figure 3), and R² for the linear equation was >0.9999.

### 3.3 Preparation and measurement of the samples to be tested

20 µL of sample (whole blood) was collected and diluted to 6 to 10 mL with 1% HNO₃, such that the concentration of Mn²⁺ ranged from 1 ppb to 100 ppb; sample IDs were generated and then the samples were measured, and Mn²⁺ concentrations of the sample solutions were calculated according to the response intensity and the standard curve.

### 3.4 Plotting of drug-time curve and calculation of pharmacokinetic parameters

PKSolver 2.0 was used for calculating relevant pharmacokinetic parameters.

The test results were shown in Figure 4 and Table 1, wherein abscissa is time (min), ordinate is Mn²⁺ concentration (ppb). The test results showed that the pharmacokinetics of KBR0826 was a non-compartment model, similar to the pharmacokinetics of extracellular fluid contrast agents. The plasma half-life of KBR0826 (*t*_{*1*/*2*} = 23 min) was close to that of gadolinium-based contrast agent.

**Table 1 Test Results of Pharmacokinetic Study**

| Parameter | Non-compartment model | Unit |
|---|---|---|
| *t*_{1/2} | 22.87±13.04 | min |
| Co | 616.61±568.85 | mg·L⁻¹ |
| AUC o-t | 712.59±325.70 | mg·min·L⁻¹ |
| AUC _{0-inf} | 734.55±333.06 | mg·min·L⁻¹ |
| AUMC _{0-inf} | 8419.63±9547.34 | mg/L·min² |
| MRT _{0-inf} | 11.26±8.93 | min |
| V | 0.38±0.45 | L· kg⁻¹ |
| CL | 0.013±0.016 | min ·L ·kg⁻¹ |
| Vss | 0.17±0.30 | L· kg⁻¹ |

### III. Excretion Kinetics Test

### 1. Experimental preparation

Clean grade Sprague Dawley (SD) rats, 5 males and 3 females, weighing 250±50 g, were purchased from the Animal Experimental Center of North Sichuan Medical College, fasted for 12-16 h before the experiment and anesthetized by intraperitoneal injection of 3% sodium pentobarbital (40 mg/kg).

### 2. Cannulation of tail vein

The rat tail was soaked in hot water at about 40 ° C for about 1 minute, and it was wiped and sterilized by a cotton ball dipped in alcohol followed by fixing and stretching the tip of the tail with left thumb and middle finger, and flatting with the index finger. The tail vein was located at both sides of the tail and was light cyan in color. Air in an indwelling needle was removed with normal saline, and the indwelling needle was inserted into the vein at an angle of 15° to the vein. When blood was returned, the indwelling needle was pushed forward after insertion. Then the indwelling needle was fixed on the tail of the rat, and normal saline was injected at a rate of about 2mL/h.

### 3. Cannulation of common bile duct

After sterilization, a longitudinal incision was made in the middle of the abdomen of rats below the xiphoid, by incising the skin, subcutaneous tissue, linea alba, and peritoneum layer by layer into the abdomen. A forcep was used for opening the left and right lobes of the liver, below which the duodenum and omentum were exposed. The transverse part of the duodenum was stretched with the forcep in the middle, and the pale yellow biliary tract was covered by the omentum and extended from the subhepatic portion to the duodenum. The biliary tract was bluntly dissected out, picked out with a glass pick, and a venous indwelling needle was inserted near the duodenum, fixed with a hemostatic clip, and bile was collected as a pre-dose sample.

### 4 Cannulation of bladder

A longitudinal incision was made at 0.5 cm above the urethral orifice of rats, and the skin was incised and the muscles were bluntly separated. After the bladder was filled to a certain extent, the bladder was lifted by the left hand with a tweezer, and the venous indwelling needle was held by the right hand and was obliquely inserted into the bladder toward the top, along the opening of ureter on the dorsal lateral side of the bladder, and the needle was removed to collect urine as a pre-dose sample.

### 5 Administration

After collecting pre-dose bile and urine samples, the drug was rapidly administered in the tail vein at a dose of 0.1mmol/kg.

### 6. Collection of bile and urine

The hourly biliary excretion amount and hourly urinary excretion amount were collected after drug administration; the samples were weighed and frozen at -20°C.

### 7. ICP-MS detection of Mn²⁺ concentration in samples

### 7.1 Preparation and detection of samples to be tested

20µL of the sample (bile and urine) was diluted to 6 to 10ml with 1% HNO₃ to make the concentrations of Mn²⁺ in the range of 1 to 1000 ppb. The concentrations of Mn²⁺ in the samples solution were numbered and detected. The Mn²⁺ concentrations in the sample solutions were calculated according to the response intensity and standard curve.

### 8. Plotting of the excretion curve

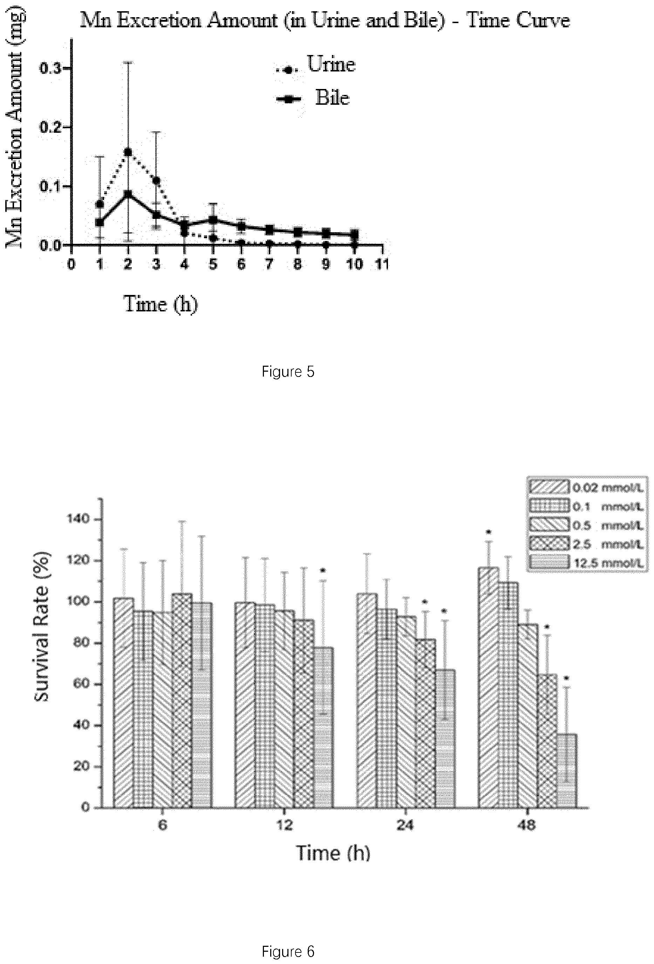

Origin 9.0 was used for calculating data and drawing. The excretion amount during the period was obtained by multiplying the dilution ratio, measurement concentration and urine (bile) volume. The excretion amount during each period was compared with the dosage of administration to obtain the percentage of cumulative drug excretion amount during the corresponding time period. According to the above data, the excretion of KBR0826 in urine and bile per unit time (hour) was shown in Table 2: excretion amount (mL), Mn²⁺ excretion amount (mg), cumulative excretion amount (mg) and cumulative excretion rate (%), and based on these data, the liver/kidney excretion curve was drawn, i.e. the time-liver/kidney excretion amount curve (Figure 5).

**Table 2 Statistics of manganese excretion amount in urine and bile of rats (n = 7)**

| Sampling period(h) | Urine | | | | Bile | | | |
|---|---|---|---|---|---|---|---|---|
| | urine amount /ml | Mn²⁺ excretion amount /mg | cumulative Mn²⁺ excretion amount /mg | cumulative Mn²⁺ excretion rate /% | bile amount/ mg | Mn²⁺ excretion amount /mg | cumulative Mn²⁺ excretion amount /mg | cumulative Mn²⁺ excretion rate /% |
| 0-1 | 0.2299 ±0.1539 | 0.06995 ±0.0808 | 0.06995 ±0.008085 | 5.28 ±5.78 | 2.3816 ±0.8613 | 0.0386 ±0.0258 | 0.0385 ±0.0258 | 2.81 ±1.53 |
| 1-2 | 0.2776 ±0.0821 | 0.15815 ±0.15147 | 0.2281 ±0.15837 | 18.27 ±13.58 | 27409 ±1.2167 | 0.0865 ±0.0657 | 0.1251 ±0.0699 | 9.31 ±4.65 |
| 2-3 | 0.3245 ±0.1271 | 0.10941 ±0.0825 | 0.33751 ±0.10837 | 26.67 ±10.50 | 2.5617 ±1.1359 | 0.0518 ±0.0199 | 0.1768 ±0.0763 | 13.17 ±4.89 |
| 3-4 | 0.2618 ±0.0986 | 0.02084 ±0.02078 | 0.33995 ±0.10653 | 26.71 ±11.14 | 2.4832 ±1.1002 | 0.0333 ±0.0146 | 0.2101 ±0.0734 | 15.69 ±4.78 |
| 4-5 | 0.2436 ±0.1046 | 0.012129 ±0.01684 | 0.35208 ±0.09321 | 27.59 ±10.28 | 2.8185 ±0.9035 | 0.043 ±0.0271 | 0.2627 ±0.0661 | 19.66 ±5.06 |
| 5-6 | 0.2327 ±0.0795 | 0.00387 ±0.00345 | 0.35595 ±0.09030 | 27.88 ±10.09 | 2.6952 ±0.7504 | 0.0318 ±0.0122 | 0.2945 ±0.0603 | 22.07 ±5.13 |
| 6-7 | 0.2427 ±0.0905 | 0.00284 ±0.00273 | 0.35879 ±0.08788 | 28.09 ±9.93 | 2.6847 ±0.7438 | 0.0265 ±0.0077 | 0.3209 ±0.0581 | 24.05 ±5.25 |
| 7-8 | 0.2082 ±0.0913 | 0.0018 ±0.00166 | 0.36059 ±0.08638 | 28.22 ±9.82 | 2.3792 ±0.7970 | 0.0219 ±0.0076 | 0.3429 ±0.0524 | 25.67 ±5.12 |
| 8-9 | 0.2261 ±0.1393 | 0.00083 ±0.00064 | 0.36143 ±0.08575 | 28.28 ±9.78 | 2.1124 ±1.1311 | 0.02± 0.0078 | 0.3684 ±0.0507 | 27.03 ±5.39 |
| 9-10 | 0.2298 ±0.0691 | 0.00061 ±0.00057 | 0.36204 ±0.08528 | 28.33 ±9.75 | 2.4402 ±1.4577 | 0.0179 ±0.0087 | 0.3859 ±0.0087 | 29.97 ±14.36 |

In Figure 5, the abscissa is time (h), and the ordinate is total metabolic amount (mg). The results showed that KBR-0826 was mainly excreted through liver and kidney, with about 60% excreted in liver and kidney within 600 min, and the liver and kidney excreted half and half. KBR-0826 was excreted in both urine and bile in rats, mainly in urine at the early stage and then in bile.

### IV. Hepatocyte toxicity test

### The experimental method

### 1. Passage, cryopreservation and resuscitation of QSG7701 cells

1) Passage: after QSG7701 cell line (25cm² culture flask) was observed to be in a good growth state, and the cell type, date and the name of culture person were marked, the QSG7701 cell line was then cultured in a 37°C incubator, with 5% CO₂. One or two days later, when the cell density meet the requirements of passage (the cell growth rate reaches 80-90%), the cells were washed 1 to 2 times with PBS, and then PBS was removed and 1mL trypsin was added to completely cover the surface of the cells for 1 to 2 minutes, followed by observing under an inverted microscope. When more than 90% of the cells have become large and round and could be shaken gently to remove a little, complete medium (DMEM medium: FBS: antibiotic= 100:10:1) was immediately added to stop digestion. The cells were blow and separated, transferred to a 15mL centrifuge tube, which was centrifuged at 1000rpm for 5min at room temperature, then the supernatant was removed. The cells were transferred to a 10MM petri dish, 5 to 10 ml fresh complete medium was added for further culture. Culture medium was changed after 2-3 days or subcultured in a ratio of 1:2 and 1:3.
2) Cryopreservation: when the cells were in the logarithmic growth phase and the growth state was good, cryopreservation was selected and a cryopreservation solution (DMEM: FBS: DMSO = 6:3:1) was prepared as above, the cells were collected after stopping digestion and centrifuging in a centrifuge tube. The cells were suspended in the cryopreservation solution, the cell concentration (1× 10⁶/mL to 10× 10⁶/mL) were adjusted and then the cells were divided into cryopreservation tubes, the cell type, date, cryopreserved person, and passage times were recorded. The tubes were placed in a gradient freezer box in a -80 °C freezer and transferred to a liquid nitrogen tank for storage the next day.
3) Cell recovery: a cryovial containing QSG7701 was taken out from the liquid nitrogen tank and put in a 37°C water bath for 1 to 2 minutes and shaken quickly to thaw. The thawed cell fluid was transferred to a 15mL centrifuge tube, centrifuged at 1000rpm for 5min at room temperature, then the supernatant was removed, and 5 to 10mL fresh complete culture medium was added into it, the cell type, date, and the name of the culture person were recorded, the cells were cultured in a 37°C incubator with 5% CO₂.

### 2 Preparation of KBR0826 solution

31.93 mg of KBR0826 solid powder was taken to prepare a 12.5 mmol/L KBR0826 solution with serum-containing medium, and the KBR0826 solution was diluted to obtain a series of solutions at a concentration of 12.5 mmol/L, 2.5 mmol/L, 0.5 mmol/L, 0.1 mmol/L, and 0.02 mmol/L, respectively, for later use;

### 3 Cell culture and administration

1) Four 96-well plates were used, each one for each detection time (6h, 12h, 24h, 48h). Each plate was configured with a control group and 5 administration groups of KBR0826 with different concentrations. Each administration group had 5 wells, and a total of 30 wells were used. 100 µL of cell fluid in logarithmic growth phase was added to each well.
2) the plate was incubated overnight (24h) in a 37°C incubator with 5% CO₂. After observing that the cells adhered well, the old medium was removed. The control group was replaced with a new serum-containing medium, and the rest were cultured respectively in 12.5 mmol/L, 2.5 mmol/L, 0.5 mmol/L, 0.1 mmol/L, and 0.02 mmol/L of KBR0826 serum-containing medium, for subculturing.
3) a 96-well plate was respectively taken out in 6h, 12h, 24h, and 48h after administration, the medium was removed, 100µL of MTT-containing serum-free medium (MTT 0.5mg/mL) was added, and then the cells were cultured in the incubator for 2h.
4) Finally, the 96-well plate was taken out, the medium was removed, and 100µl DMSO was added to the plate, shaken horizontally and mixed for 2~3 minutes to fully dissolve the crystals, and the OD value was read at the detection line of 595nm with a microplate reader.

### 4 Statistical Methods

Origin 9.0 software was used for data mapping, and measurement data were expressed as mean ± standard deviation (±sd); SPSS14.0 statistical software was used for data statistics, and univariate analysis was used.

### 5 Results

The results of the survival rate (595nm) of normal human hepatocytes in various concentrations of KBR0826 at different time are shown in Figure 6. The statistical analysis results of adding different concentrations of KBR0826 for cell culture show that when the concentrations of KBR0826 (0.02 mmol /L, 0.1 mmol /L, or 0.5 mmol /L) are lower, the survival rate of the QSG7701 hepatocyte line at each detection time point (6h, 12h, 24h, 48h) does not decrease significantly; but when the concentration of KBR0826 is 2.5 mmol/L, the survival rates of the QSG7701 hepatocyte line detected at 24h and 48h decrease significantly (p < 0.05), and the survival rate of the QSG7701 hepatocyte cell line decreases significantly at 12 h when the concentration of KBR0826 is 12.5 mmol/L (p < 0.05). Within 24 hours, KBR0826 in 25 times of the clinical dose (2.5 mmol/L) has non-obvious inhibitory effect on the proliferation of QSG7701 hepatocyte line.

Wherein, ^{∗} indicates a statistical difference after comparing with the OD value of the control group.

### V. In vivo imaging in rats

1. Sixteen SD rats were divided into a KBR0826 contrast agent group (experimental group) and a gadoteric acid meglumine (Gd-DOTA) contrast agent group (control group), with 8 rats in each group, fasted for 12 hours (drank water freely).
2. 3% sodium pentobarbital (60 to 70mg/kg) was weighed for intraperitoneal anesthesia.
3. The dosage of KBR0826 was 0.2 mmol/kg. SD rats were imaged in vivo with a 3.0 T MRI imager. Contrast agents were injected into the tail vein. The signal intensities (SI) of the heart, liver, brain parenchyma, muscle, and abdominal aorta in rats were measured before and after injection, and signal to noise ratio (SNR) was calculated and time-signal intensity curve was drawn to evaluate the imaging effect of the contrast agents.

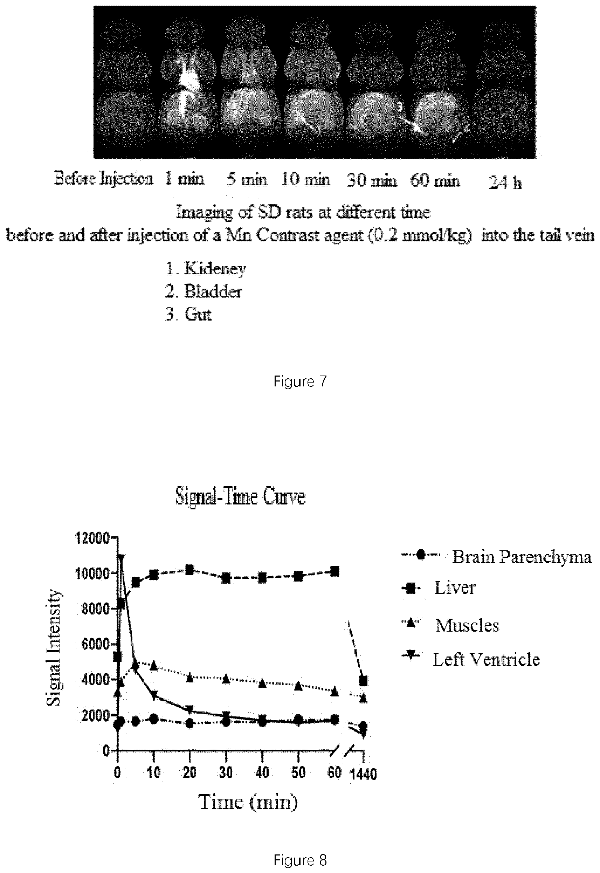

The imaging effect was shown in Figure 7. The result showed that the changing of the cardiovascular signal intensity of KBR-0826 is similar to that of Gd-DOTA, and the liver enhancement is obvious and continuous; the imaging signal intensity of KBR-0826 in the rat organs was shown in Figure 8, wherein the abscissa is the time , and the ordinate is the signal intensity. The results showed that KBR-0826 could not pass through the normal blood-brain barrier and could be excreted through the kidneys and intestines.

To sum up, the above test results show that the paramagnetic manganese complex magnetic resonance imaging contrast agent of the Example has the characteristics of good water solubility, high relaxivity, and low toxicity and side effects, and has the prospect of being applied as an MRI contrast agent in clinic.

### Example 4

The compound obtained in step (4) in Example 1 (3.60 g, 8.6 mmol) was added to 30 mL of pure water, anhydrous FeCl₃ (1.38 g, 8.5 mmol) was added under stirring, and the pH was adjusted to 7.3 to 7.4 with solid sodium hydroxide, to obtain a solution which was freeze-dried into a 5.5 g solid.

The relaxation efficiencies of the above complexes were measured and the results are as follows: *r₁* = 1.80 mmol⁻¹s⁻¹, *r₂* = 2.10 mmol⁻¹s⁻¹ (0.5 T, 32 °C).

The technical features of the above embodiments can be combined arbitrarily. For the sake of brief description, not all possible combinations of the technical features in the above embodiments are described. However, the combination of these technical features should be considered within the scope of the present specification as long as they are not contradictory.

The above embodiments only express several implementation modes of the present invention, which are described concretely and detailly and cannot be understood as a limitation of the invention patent scope. It should be pointed out that, for ordinary one skilled in the art, several modifications and improvements can also be made, which are within the protection scope of the invention, without departing from the contents of the present disclosure. Therefore, the protection scope of the invention patent shall be subject to the appended claims.

## Claims

1. A compound having a structure of formula I, or a pharmaceutically acceptable salt thereof,

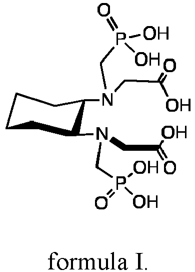

2. A method of preparing the compound having a structure of formula I of claim 1, wherein a synthetic scheme is as follows: 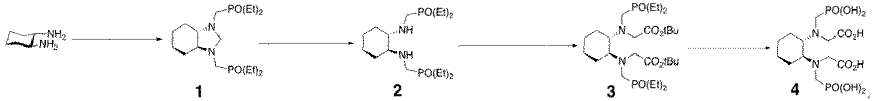
S1: condensing paraformaldehyde and phosphinate through a Kabachnick-Fields reaction with a raw material cyclohexanediamine, to obtain Compound 1;
S2: hydrolyzing the Compound 1 to obtain Compound 2 in an acid;
S3: N-alkylating Compound 2 to obtain Compound 3;
S4: hydrolyzing the Compound 3 to obtain Compound 4, i.e. the compound having a structure of formula I.

3. The method of claim 2, wherein,
in S1, cyclohexanediamine, diethyl phosphite and paraformaldehyde are added into a solvent tetrahydrofuran, and mixed and refluxed to obtain the Compound 1;
in S2, methanol is added to the compound obtained in the S1 and reacted in a high concentration of acid to obtain Compound 2;
in S3, the compound obtained in the S2 is dissolved into acetonitrile, then KI, tert-butyl bromoacetate and alkali are added and reacted;
in S4, the compound obtained in the S3 is dissolved into an acid solution, heated with reflux, and a protecting group is removed to further obtain Compound 4, i.e. the compound having a structure of formula I.

4. A use of the compound having a structure of formula I or a pharmaceutically acceptable salt thereof of claim 1 in a preparation of a magnetic resonance imaging contrast agent.

5. The use of claim 2, wherein the compound having a structure of formula I is used as a ligand in the preparation of magnetic resonance imaging contrast agent.

6. A compound having a structure of formula II or, a pharmaceutically acceptable salt thereof, 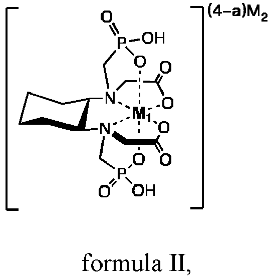 wherein,
M₁ is a divalent ion and selected from Mn, Fe, Eu or Dy, or M₁ is a trivalent ion and selected from Mn, Fe, Eu or Dy; and
M₂ is selected from Na⁺, K⁺ or meglumine cation;
when M₁ is a divalent ion, a is 2; when M₁ is a trivalent ion, a is 3.

7. The compound of claim 6, wherein, M₁ is Mn²⁺ or Fe ³⁺; M₂ is Na⁺.

8. A method of preparing the compound having a structure of formula II of claim 6, wherein the compound having the structure of formula I or a pharmaceutically acceptable salt thereof reacts with compound having an M₁ ion, and alkali is added for regulating pH in a range of 6 to 9, thereby obtaining the compound having a structure of formula II.

9. The method of claim 8, wherein, alkali is sodium hydroxide, potassium hydroxide, or meglumine.

10. A use of the compound having a structure of formula II of claim 6 or claim 7 in a preparation of a magnetic resonance imaging contrast agent.

11. A magnetic resonance imaging contrast agent, comprising the compound having a structure of formula II and a pharmaceutically acceptable excipient of claim 6 or claim 7.
